(19) Europäisches Patentamt
European Patent Office
Office européen des brevets

(11) Veröffentlichungsnummer: **0 433 779 B1**

(12) # EUROPÄISCHE PATENTSCHRIFT

(45) Veröffentlichungstag der Patentschrift: **22.02.95**

(51) Int. Cl.[6]: **C07D 251/46**, C07D 251/16, A01N 43/66

(21) Anmeldenummer: **90123370.0**

(22) Anmeldetag: **06.12.90**

Die Akte enthält technische Angaben, die nach dem Eingang der Anmeldung eingereicht wurden und die nicht in dieser Patentschrift enthalten sind.

(54) **Verfahren zur Herstellung von Sulfonylharnstoff-Salzen und ihre Verwendung als Herbizide.**

(30) Priorität: **19.12.89 DE 3941790**
**08.06.90 DE 4018349**

(43) Veröffentlichungstag der Anmeldung:
**26.06.91 Patentblatt 91/26**

(45) Bekanntmachung des Hinweises auf die Patenterteilung:
**22.02.95 Patentblatt 95/08**

(84) Benannte Vertragsstaaten:
**BE CH DE ES FR GB IT LI NL**

(56) Entgegenhaltungen:
**EP-A- 0 251 079**
**EP-A- 0 304 282**
**DE-A- 3 609 700**

(73) Patentinhaber: **BAYER AG**

**D-51368 Leverkusen (DE)**

(72) Erfinder: **Riebel, Hans-Jochem, Dr.**
**In der Beek 92**
**W-5600 Wuppertal 1 (DE)**
Erfinder: **Fest, Christa, Dr.**
**Im Johannistal 20**
**W-5600 Wuppertal 1 (DE)**
Erfinder: **Kirsten, Rolf, Dr.**
**Carl-Langhans-Strasse 27**
**W-4019 Monheim (DE)**
Erfinder: **Kluth, Joachim, Dr.**
**Tannenweg 9**
**W-4019 Langenfeld (DE)**
Erfinder: **Müller, Klaus-Helmut, Dr.**
**Bockhackstrasse 55**
**W-4000 Düsseldorf 13 (DE)**
Erfinder: **Feucht, Dieter, Dr.**
**Geschwister-Scholl-Strasse 88**
**W-4019 Monheim 2 (DE)**
Erfinder: **Lürssen, Klaus, Dr.**
**August-Kierspel-Strasse 145**
**W-5060 Bergisch Gladbach 2 (DE)**
Erfinder: **Santel, Hans-Jochim, Dr.**
**Grünstrasse 9a**
**W-5090 Leverkusen 1 (DE)**
Erfinder: **Schmidt, Robert R., Dr.**
**Im Waldwinkel 110**
**W-5060 Bergisch Gladbach 2 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Die Erfindung betrifft ein neues Verfahren zur Herstellung von Sulfonylharnstoff-Salzen, neue Sulfonylharnstoff-Salze und ihre Verwendung als Herbizide,

Es ist bereits bekannt, daß man Alkalimetallsalze von substituierten N-Phenylsulfonyl-N'-(allyl- oder -propargyl)-N'-azinyl-harnstoffen (A), welche eine herbizide und pflanzenwuchsregulierende Wirkung haben, herstellen kann, indem man die Sulfonylharnstoffe (A) unter Stickstoff mit einem Alkalimethylat, gelöst in absolutem Methanol, umsetzt (vgl. DE-A- 3 609 700; Beispiele 1-11).

Ferner ist bekannt, daß man Sulfonylharnstoff-Salze erhält, wenn man Lösungen von Sulfonylharnstoffen in Halogenkohlenwasserstoff-Solventien mit Alkalimetall- oder Erdalkalimetall-hydroxiden umsetzt und, gegebenenfalls nach Filtration, das Lösungsmittel abdestilliert (vgl. EP-A 304 282).

Da bei dieser Verfahrensweise im allgemeinen keine Kristallisation des Produktes aus der Lösung erfolgt, ist eine energieaufwendige Destillation des Lösungsmittels erforderlich und die Chance, einen Reinigungseffekt zu erzielen, kaum gegeben.

3-Substituierte-1-(2-Halogenalkoxy-benzolsulfonyl)-3-heteroaryl-(thio)harnstoffe sind als Herbizide bekannt (vgl. EP-A 251 079). Diese Verbindungen werden jedoch im wäßrigen Milieu relativ rasch hydrolytisch zersetzt. Salze dieser Verbindungen, die eine höhere Stabilität besitzen sollten, konnten bisher noch nicht in genügender Qualität hergestellt werden.

Es wurde nun ein Verfahren zur Herstellung von Sulfonylharnstoff-Salzen der allgemeinen Formel (I)

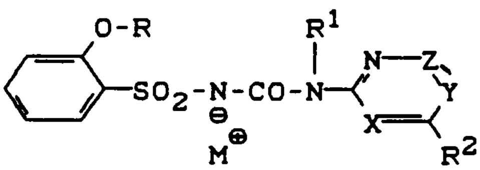

(I)

in welcher

- $M^{\oplus}$ für ein Alkalimetallion oder das durch Protonierung gebildete Gegenion einer basischen organischen Stickstoffverbindung steht,
- R für Halogenalkyl steht,
- $R^1$ für gegebenenfalls substituierte Reste aus der Reihe Alkyl, Alkenyl, Alkinyl oder Aralkyl steht,
- $R^2$ für Wasserstoff, Halogen, Alkyl, Halogenalkyl, Alkoxy, Halogenalkoxy, Alkylthio, Halogenalkylthio, Amino, Alkylamino oder Dialkylamino steht,
- X für Stickstoff oder eine -CH-Gruppierung steht,
- Y für Stickstoff oder eine $-CR^3$-Gruppierung steht, worin
- $R^3$ für Wasserstoff, Halogen, Cyano, Alkyl, Formyl, Alkyl-carbonyl oder Alkoxy-carbonyl steht und
- Z für Stickstoff oder eine $-CR^4$-Gruppierung steht, worin
- $R^4$ für Wasserstoff, Halogen, Alkyl, Halogenalkyl, Alkoxy, Halogenalkoxy, Alkylthio, Alkylamino oder Dialkylamino steht,

gefunden, welches dadurch gekennzeichnet ist, daß man Sulfonylharnstoffe der allgemeinen Formel (II)

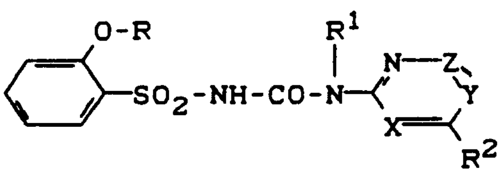

(II)

in welcher

R, $R^1$, $R^2$, X, Y und Z die oben angegebenen Bedeutungen haben,

mit Alkalimetallhydroxiden oder mit basischen organischen Stickstoffverbindungen in Gegenwart von Kohlenwasserstoffen als Verdünnungsmitteln bei Temperaturen zwischen schen 0 °C und 100 °C umsetzt und die hierbei kristallin anfallenden Produkte nach üblichen Methoden isoliert.

Weiter wurde gefunden, daß die nach dem erfindungsgemäßen Verfahren herzustellenden Sulfonylharnstoff-Salze der Formel (I) sich durch starke herbizide Wirksamkeit auszeichnen.

Gegenstand der vorliegenden Erfindung sind weiterhin die beiden neuen, unter die obige Formel (I) fallenden Verbindungen 3-(4,6-Dimethoxy-s-triazin-2-yl)-3-methyl-1-(trifluormethoxy-phenylsulfonyl)-harnstoff-Natriumsalz und 3-(4,6-Dimethoxy-s-triazin-2-yl)-3-methyl-1-(2-di= fluormethoxy-phenylsulfonyl)-harnstoff-Natriumsalz, welche sich auch durch die Formel (Ia)

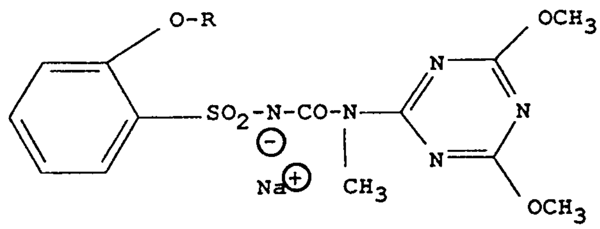

(Ia)

in welcher R für $CF_3$ oder $CHF_2$ steht,
beschreiben lassen.

Es ist als überraschend anzusehen, daß man nach dem erfindungsgemäßen Verfahren die Sulfonylharnstoff-Salze der Formel (I) auf sehr einfache Weise in sehr guten Ausbeuten und in hoher Reinheit erhält, da eine glatte Umsetzung der polaren Reaktionskomponenten in Kohlenwasserstoffen als unpolaren Lösungsmitteln nicht zu erwarten war.

Wie vergleichende Versuche zudem gezeigt haben, wird bei analoger Umsetzung in polaren Lösungsmitteln ein mehr oder weniger großer Anteil an Zersetzungsprodukten gebildet, die nach Umsetzung gemäß dem erfindungsgemäßen Verfahren praktisch nicht auftreten.

Die neuen Sulfonylharnstoff-Salze zeigen gegenüber den entsprechenden "freien" Sulfonylharnstoffen insbesondere die vorteilhafte Eigenschaft, daß ihre Lagerungs- und Anwendungsformen (Formulierungen und Spritzbrühen) wesentlich stabiler sind.

Das erfindungsgemäße Verfahren betrifft vorzugsweise die Herstellung von Sulfonylharnstoff-Salzen der Formel (I), in welcher

$M^{\oplus}$ für ein Lithium-, Natrium- oder Kaliumion, für ein Tri-($C_1$-$C_4$-alkyl)-ammoniumion, für ein N-($C_3$-$C_6$-Cycloalkyl)-N,N-di-($C_1$-$C_4$-alkyl)-ammoniumion oder jeweils für das durch Protonierung gebildete Gegenion von N-($C_1$-$C_4$-Alkyl)-pyrrolidin, N-($C_1$-$C_4$-Alkyl)-piperidin, N-($C_1$-$C_4$-Alkyl)-morpholin, N, N'-Di-($C_1$-$C_4$-alkyl)-piperazin, N,N-Di-($C_1$-$C_4$-alkyl)-benzylamin, 1,5-Diazabicyclo-[4.3.0]-non-5-en (DBN), 1,8-Diazabicyclo-[5.4.0]-undec-7-en (DBU) oder 1,4-Diazabicyclo-[2.2.2]-octan [DABCO] steht,

R für Halogen-$C_1$-$C_4$-alkyl steht,

$R^1$ für $C_1$-$C_6$-Alkyl (welches gegebenenfalls durch Fluor, Cyano, $C_1$-$C_4$-Alkoxy oder $C_1$-$C_4$-Alkylthio substituiert ist), für $C_3$-$C_6$-Alkenyl und $C_3$-$C_6$-Alkinyl (welche gegebenenfalls durch Fluor oder Chlor substituiert sind) oder für Phenyl-$C_1$-$C_2$-alkyl (welches im Phenylteil gegebenenfalls durch Fluor, Chlor, Nitro, Cyano, Methyl, Methoxy oder $C_1$-$C_2$-Alkoxycarbonyl substituiert ist) steht,

$R^2$ für Wasserstoff, Fluor, Chlor, Brom, Methyl, Ethyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine -CH-Gruppierung steht,

Y für Stickstoff oder eine -$CR^3$-Gruppierung steht, worin

$R^3$ für Wasserstoff, Fluor, Chlor, Brom, Methyl, Formyl, Acetyl, Methoxycarbonyl oder Ethoxycarbonyl steht,

und

Z für Stickstoff oder eine -$CR^4$-Gruppierung steht, worin

$R^4$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht.

Das erfindungsgemäße Verfahren betrifft insbesondere die Herstellung von Sulfonylharnstoff-Salzen der Formel (I),
in welcher

$M^{\oplus}$ für ein Natriumion, ein Kaliumion, für ein Trimethyl-, Triethyl-, Tripropyl- oder Tributylammoniumion, für ein N,N-Dimethyl-, N,N-Diethyl- oder N,N-Dipropyl-cyclopentylammoniumion, für ein N,N-Dimethyl-, N,N-Diethyl- oder N,N-Dipropyl-cyclohexylammoniumion, oder jeweils für das

durch Protonierung gebildete Gegenion von N-Methyl-, N-Ethyl- oder N-Propyl-pyrrolidin, N-Methyl-, N-Ethyl- oder N-Propyl-piperidin, N-Methyl-, N-Ethyl- oder N-Propyl-morpholin, N,N'-Dimethyl-, N,N'-Diethyl- oder N,N'-Dipropyl-piperazin, N,N-Dimethyl-, N,N-Diethyl- oder N,N-Dipropyl-benzylamin, 1,5-Diazabicyclo-[4.3.0]-non-5-en (DBN), 1,8-Diazabicyclo-[5.4.0]-undec-7-en (DBU) oder 1,4-Diazabicyclo-[2.2.2]-octan (DABCO) steht,

R für Difluormethyl oder Trifluormethyl steht,

$R^1$ für Methyl steht,

$R^2$ für Wasserstoff, Chlor, Methyl, Ethyl, Trifluormethyl, Methoxy, Ethoxy, Difluormethoxy, Methylthio, Ethylthio, Methylamino, Ethylamino oder Dimethylamino steht,

X für Stickstoff steht,

Y für Stickstoff oder eine CH-Gruppierung (ganz besonders bevorzugt für Stickstoff) steht und

Z für eine C-$R^4$-Gruppierung steht, worin

$R^4$ für Wasserstoff, Chlor, Methyl, Ethyl, Trifluormethyl, Methoxy, Ethoxy, Difluormethoxy, Methylthio, Ethylthio, Methylamino, Ethylamino oder Dimethylamino steht.

Verwendet man beispielsweise 3-(4,6-Dimethoxy-pyrimidin-2-yl)-3-methyl-1-(2-difluormethoxy-phenylsulfonyl)-harnstoff und Trimethylamin als Ausgangsstoffe, so kann der Reaktionsablauf beim erfindungsgemäßen Verfahren durch das folgende Formelschema skizziert werden:

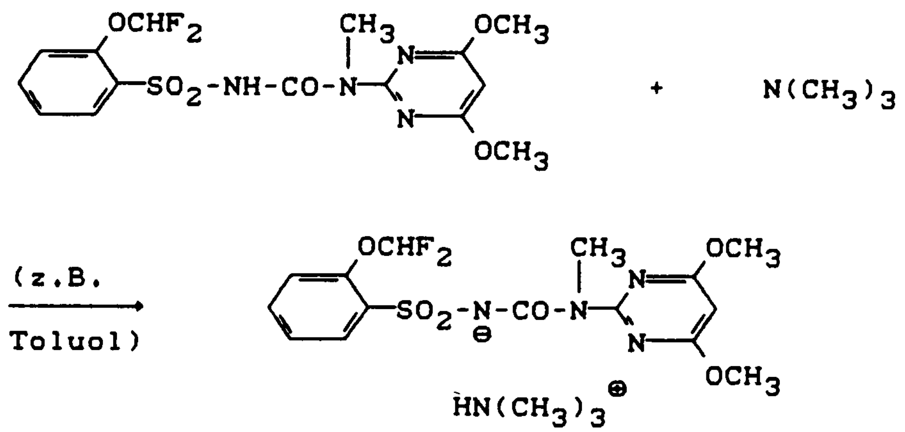


Die beim erfindungsgemäßen Verfahren zur Herstellung von Verbindungen der Formel (I) als Ausgangsstoffe zu verwendenden Sulfonylharnstoffe sind durch die Formel (II) allgemein definiert.

In Formel (II) haben R, $R^1$, $R^2$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R, $R^1$, $R^2$, X, Y und Z angegeben wurden.

Beispiele für die Ausgangsstoffe der Formel (II) sind in der nachstehenden Tabelle 1 aufgeführt.

Tabelle 1: Beispiele für die Ausgangsstoffe der Formel (II)

| R | $R^1$ | $R^2$ | X | Y | Z |
|---|---|---|---|---|---|
| $CF_3$ | $CH_3$ | $OCH_3$ | N | CH | $C-OCH_3$ |
| $CF_3$ | $CH_3$ | $CH_3$ | N | N | $C-OCH_3$ |
| $CF_3$ | $CH_3$ | $OCH_3$ | N | CH | C-Cl |
| $CF_3$ | $CH_3$ | $C_2H_5$ | N | CH | $C-OCH_3$ |
| $CF_3$ | $CH_3$ | $CH_3$ | N | CH | $C-OC_2H_5$ |
| $CF_3$ | $CH_3$ | $CH_3$ | N | CH | $C-OCH_3$ |
| $CF_3$ | $CH_3$ | $CH_3$ | N | CH | $C-CH_3$ |
| $CF_3$ | $CH_3$ | $CH_3$ | N | CH | C-Cl |
| $CF_3$ | $CH_3$ | $CF_3$ | N | CH | $C-OCH_3$ |
| $CHF_2$ | $CH_3$ | $OCH_3$ | N | CH | C-Cl |
| $CHF_2$ | $CH_3$ | $OCH_3$ | N | CH | $C-OCH_3$ |
| $CHF_2$ | $CH_3$ | $CH_3$ | N | N | $C-CH_3$ |
| $CHF_2$ | $CH_3$ | $CH_3$ | N | N | $C-OCH_3$ |

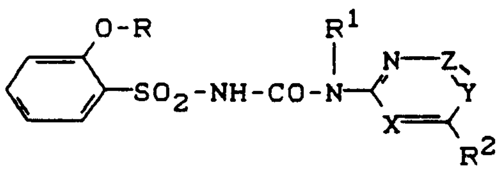

(II)

Tabelle 1 - Fortsetzung

| R | $R^1$ | $R^2$ | X | Y | Z |
|---|---|---|---|---|---|
| $CHF_2$ | $CH_3$ | $OCH_3$ | N | N | $C-OCH_3$ |
| $CHF_2$ | $CH_3$ | $CF_3$ | N | CH | $C-OCH_3$ |
| $CF_3$ | $CH_3$ | $CH_3$ | N | N | $C-CH_3$ |
| $CF_3$ | $CH_3$ | $C_2H_5$ | N | N | $C-OCH_3$ |
| $CF_3$ | $CH_3$ | $CH_3$ | N | N | $C-OC_2H_5$ |
| $CF_3$ | $CH_3$ | $OCH_3$ | N | N | $C-OCH_3$ |
| $CF_3$ | $CH_3$ | $OC_2H_5$ | N | N | $C-OC_2H_5$ |
| $CF_3$ | $CH_3$ | $OCH_3$ | N | N | $C-NHCH_3$ |
| $CF_3$ | $CH_3$ | $OCH_3$ | N | N | $C-NHC_2H_5$ |
| $CF_3$ | $CH_3$ | $OC_2H_5$ | N | N | $C-NHCH_3$ |
| $CF_3$ | $CH_3$ | $CH_3$ | N | N | $C-SCH_3$ |
| $CF_3$ | $CH_3$ | $OCH_3$ | N | N | $C-SCH_3$ |

Die Ausgangsstoffe der Formel (II) sind bekannt und/oder können nach an sich bekannten Verfahren hergestellt werden (vgl. EP-A 251 079).

Das erfindungsgemäße Verfahren wird unter Einsatz von Alkalimetallhydroxiden oder von basischen organischen Stickstoffverbindungen durchgeführt. Bevorzugt werden Lithium-, Natrium- und Kalium-hydroxid, Tri-($C_1$-$C_4$-alkyl)-amine, N-($C_3$-$C_6$-Cycloalkyl)-N,N-di-($C_1$-$C_4$-alkyl)-amine, N-($C_1$-$C_4$-Alkyl)-pyrrolidine, N-($C_1$-$C_4$-Alkyl)-piperidine, N-($C_1$-$C_4$-Alkyl)-morpholine, N,N'-Di-($C_1$-$C_4$-alkyl)-piperazine, N,N-Di-($C_1$-$C_4$-alkyl)-benzylamine, 1,5-Diazabicyclo-[4.3.0]-non-5-en (DBN), 1,8-Diazabicyclo-[5.4.0]-undec-7-en (DBU) und 1,4-Diazabicyclo-[2.2.2]-octan (DABCO).

Besonders bevorzugt werden Natrium- und Kalium-hydroxid, Trimethyl-, Triethyl-, Tripropyl- und Tributylamin, N,N-Dimethyl-, N,N-Diethyl- und N,N-Dipropyl-cyclopentylamin, N,N-Dimethyl-, N,N-Diethyl- und N,N-Dipropyl-cyclohexylamin, N-Methyl-, N-Ethyl- und N-Propyl-pyrrolidin, N-Methyl-, N-Ethyl- und N-Propyl-piperidin, N-Methyl-, N-Ethyl- und N-Propyl-morpholin, N,N'-Dimethyl-, N,N'-Diethyl- und N,N'-Dipropyl-piperazin, N,N-Dimethyl-, N,N-Diethyl- und N,N-Dipropyl-benzylamin, 1,5-Diazabicyclo-[4.3.0]-non-5-en (DBN), 1,8-Diazabicyclo-[5.4.0]-undec-7-en (DBU) und 1,4-Diazabicyclo-[2.2.2]-octan (DABCO).

Das erfindungsgemäße Verfahren wird in Gegenwart von Kohlenwasserstoffen als Verdünnungsmitteln durchgeführt. Als Verdünnungsmittel beim erfindungsgemäßen Verfahren werden aromatische, insbesondere benzoide Kohlenwasserstoffe bevorzugt, welche gegebenenfalls 1 bis 3 Alkyl-substituenten mit jeweils 1 bis 3 Kohlenstoffatomen enthalten. Als Beispiele hierfür seien genannt: Benzol, Toluol, o-, m- und p-Xylol, Ethylbenzol, Propylbenzol, Cumol, 1,2,3-Trimethylbenzol, 1,2,4-Trimethylbenzol und 1,3,5-Trimethylbenzol. Toluol wird als Verdünnungsmittel beim erfindungsgemäßen Verfahren ganz besonders bevorzugt.

Die Reaktionstemperaturen können bei dem erfindungsgemäßen Verfahren in einem größeren Bereich variiert werden. Im allgemeinen arbeitet man bei Temperaturen zwischen 0 °C und 100 °C, vorzugsweise bei Temperaturen zwischen 10 °C und 50 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Sulfonylharnstoff der Formel (I) im allgemeinen zwischen 0,9 und 1,5 Mol, vorzugsweise zwischen 1,0 und 1,3 Mol, eines Alkalimetallhydroxids oder einer basischen organischen Stickstoffverbindung ein.

Die Reaktionskomponenten können in beliebiger Reihenfolge zusammengegeben werden. In einer bevorzugten Ausführungsform des erfindungsgemäßen Verfahrens wird der Sulfonylharnstoff der Formel (II) zunächst mit dem Kohlenwasserstoff-Verdünnungsmittel verrührt und dann mit einem Alkalimetallhydroxid oder einer basischen organischen Stickstoffverbindung versetzt. Das Reaktionsgemisch wird gerührt, bis die Kristallisation des Produktes praktisch abgeschlossen ist; anschließend wird das Produkt durch Absaugen isoliert.

Die erfindungsgemäßen Wirkstoffe können als Defoliants, Desiccants, Krautabtötungsmittel und insbesondere als Unkrautvernichtungsmittel verwendet werden, Unter Unkraut im weitesten Sinne sind alle Pflanzen zu verstehen, die an Orten aufwachsen, wo sie unerwünscht sind, Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Verbindungen eignen sich insbesondere zur selektiven Bekämpfung von dikotylen Unkräutern in monokotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOP); N,H-Di-n-propylthiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acet amid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 2-Chlor-N-isopropylacetanilid (PROPACHLOR); 0-(6-Chlor-3-phenylpyridazin-4-yl)-S-octyl-thiolcarbamat (PYRIDATE); 2-[4-(6-Chlor-chinoxalin-2-yl-oxy)-phenoxy]-propionsäure-ethylester (QUIZALOFOPETHYL); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 1000 g Wirkstoff pro Hektar Bodenfläche, vorzugsweise zwischen 5 g und 500 g pro ha.

Die Herstellung und die Verwendung der erfindungsgemäßen Wirkstoffe geht aus den nachfolgenden Beispielen hervor.

Herstellungsbeispiele:

Beispiel 1

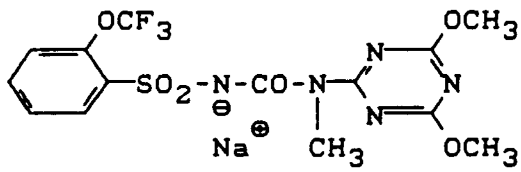

4,4 g (0,010 Mol) 3-(4,6-Dimethoxy-s-triazin-2-yl)-3-methyl-1-(2-trifluormethoxy-phenylsulfonyl)-harnstoff werden mit 15 ml Toluol verrührt und dann mit 0,5 g (0,0125 Mol) Natriumhydroxid-Pulver versetzt. Das Reaktionsgemisch wird 60 Minuten bei 20 °C bis 25 °C gerührt, wobei ein zäher Kristallbrei entsteht, der durch Zugabe von insgesamt 30 ml Toluol rührbar gehalten wird. Nach Ende der Umsetzung wird das kristalline Produkt durch Absaugen isoliert.

Man erhält 4,0 g (87% der Theorie) 3-(4,6-Dimethoxy-s-triazin-2-yl)-3-methyl-1-(2-trifluormethoxy-phenylsulfonyl)-harnstoff-Natriumsalz; Schmelzpunkt 224 °C.

Beispiel 2

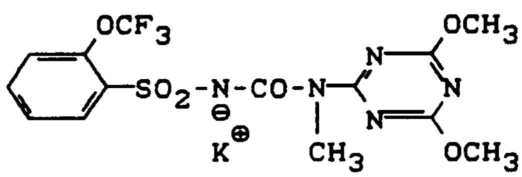

4,4 g (0,010 Mol) 3-(4,6-Dimethoxy-s-triazin-2-yl)-3-methyl-1-(2-trifluormethoxy-phenylsulfonyl)-harnstoff werden mit 50 ml Toluol verrührt und dann mit 0,8 g 80%igem Kaliumhydroxid-Pulver (0,0114 Mol KOH) versetzt. Das Reaktionsgemisch wird 15 Stunden bei 20 °C bis 25 °C gerührt und das kristallin angefallene Produkt durch Absaugen isoliert.

Man erhält 4,1 g (86% der Theorie) 3-(4,6-Dimethoxy-s-triazin-2-yl)-3-methyl-1-(2-trifluormethoxy-phenylsulfonyl)-harnstoff-Kaliumsalz; Schmelzpunkt 193 °C (Zers.).

Analog können beispielsweise auch die in der nachstehenden Tabelle 2 aufgeführten Verbindungen der Formel (I) hergestellt werden.

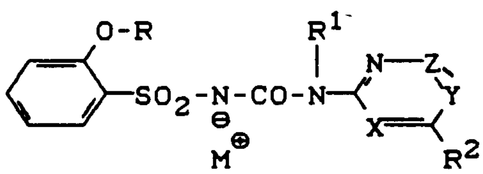

(I)

<u>Tabelle 2:</u> Beispiele für die Verbindungen der Formel (I)

| Bsp.-Nr. | R | $R^1$ | $R^2$ | X | Y | Z | $M^{\oplus}$ | Schmelzpunkt (°C) |
|---|---|---|---|---|---|---|---|---|
| 3 | $CF_3$ | $CH_3$ | $CH_3$ | N | N | $C-CH_3$ | $Na^{\oplus}$ | 98 |
| 4 | $CF_3$ | $CH_3$ | $CH_3$ | N | N | $C-OCH_3$ | $Na^{\oplus}$ | >240 (Zers.) |
| 5 | $CF_3$ | $CH_3$ | $OCH_3$ | N | N | $C-OCH_3$ | $(C_2H_5)_3NH^{\oplus}$ | 125 (Zers.) |
| 6 | $CHF_2$ | $CH_3$ | $CH_3$ | N | N | $C-OCH_3$ | $Na^{\oplus}$ | 250 |
| 7 | $CHF_2$ | $CH_3$ | $OCH_3$ | N | N | $C-OCH_3$ | $Na^{\oplus}$ | 205 |
| 8 | $CF_3$ | $CH_3$ | $OCH_3$ | N | N | $C-OCH_3$ | $(CH_3)_3NH^{\oplus}$ | 119 |
| 9 | $CF_3$ | $CH_3$ | $OCH_3$ | N | N | $C-OCH_3$ | 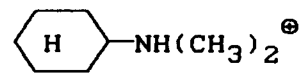 | 82 |
| 10 | $CF_3$ | $CH_3$ | $OCH_3$ | N | N | $C-OCH_3$ | 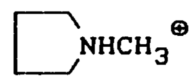 | 114 |

Tabelle 2: Beispiele für die Verbindungen der Formel (I)

| Bsp.-Nr. | R | $R^1$ | $R^2$ | X | Y | Z | $M^{\oplus}$ | Schmelzpunkt (°C) |
|---|---|---|---|---|---|---|---|---|
| 11 | $CF_3$ | $CH_3$ | $OCH_3$ | N | N | $C-OCH_3$ | 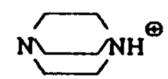 | 121 |
| 12 | $CF_3$ | $CH_3$ | $OCH_3$ | N | N | $C-OCH_3$ | 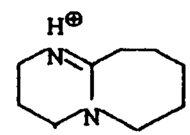 | 108 |
| 13 | $CF_3$ | $CH_3$ | $CH_3$ | N | N | $C-OCH_3$ | $K^{\oplus}$ | 200 |
| 14 | $CF_3$ | $CH_3$ | $CH_3$ | N | N | $C-OCH_3$ | 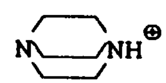 | 135 |

Die in Tabelle 2 als Beispiel 5 aufgeführte Verbindung kann beispielsweise wie folgt hergestellt werden:

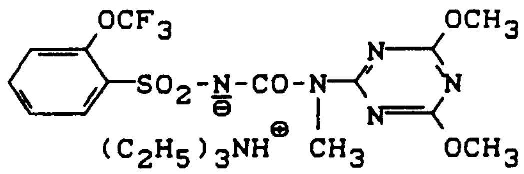

8,8 g (0,020 Mol) 3-(4,6-Dimethoxy-s-triazin-2-yl)-3-methyl-1-(2-trifluormethoxy-phenylsulfonyl)-harnstoff werden in 88 ml Toluol durch Erwärmen auf ca. 40 °C gelöst; anschließend werden bei 35 °C bis 40 °C 2,2 g (0,021 Mol) Triethylamin dazugegeben. Nach einigen Minuten beginnt die Kristallisation des Produktes. Die Mischung wird noch ca. 15 Stunden bei 20 °C gerührt, dann wird das kristalline Produkt durch Absaugen isoliert.

Man erhält 8,8 g (82% der Theorie) 3-(4,6-Dimethoxy-s-triazin-2-yl)-3-methyl-1-(2-trifluormethoxy-phenylsulfonyl)-harnstoff-Triethylammoniumsalz; Schmelzpunkt: 125 °C (Zers.).

Anwendungsbeispiele:

Beispiel A

Post-emergence-Test

Lösungsmittel: 5 Gewichtsteile Dimethylformamid
Emulgator: 1 Gewichtsteil Alkylarylpolyglykolether
Additiv: 0,1% Renex-36 (= Polyoxyethylen-(6)-tri-decylether; Netzmittel)

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator und Additiv zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden, Die Konzentration der Spritzbrühe wird so gewählt, daß in 1000 l Wasser/ha die jeweils gewünschten Wirkstoffmengen ausgebracht werden, Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 1, 2, 4, 5, 8, 9, 10, 11 und 12 bei sehr guter Verträglichkeit gegenüber Weizen, Gerste und Mais sehr starke Wirkung gegen Unkräuter.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei halt man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 1, 2, 4 und 5 bei sehr guter Verträglichkeit gegenüber Weizen, Gerste und Mais sehr starke Wirkung gegen Unkräuter.

## Patentansprüche

**1.** Verfahren zur Herstellung von Sulfonylharnstoff-Salzen der allgemeinen Formel (I)

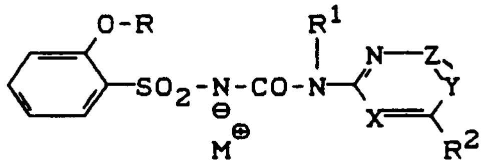

(I)

in welcher

$M^{\oplus}$ für ein Alkalimetallion oder das durch Protonierung gebildete Gegenion einer basischen organischen Stickstoffverbindung steht,

R für Halogenalkyl steht,

$R^1$ für gegebenenfalls substituierte Reste aus der Reihe Alkyl, Alkenyl, Alkinyl oder Aralkyl steht,

$R^2$ für Wasserstoff, Halogen, Alkyl, Halogenalkyl, Alkoxy, Halogenalkoxy, Alkylthio, Halogenalkylthio, Amino, Alkylamino oder Dialkylamino steht,

X für Stickstoff oder eine -CH-Gruppierung steht,

Y für Stickstoff oder eine $-CR^3$-Gruppierung steht, worin

$R^3$ für Wasserstoff, Halogen, Cyano, Alkyl, Formyl, Alkyl-carbonyl oder Alkoxycarbonyl steht und

Z für Stickstoff oder eine $-CR^4$-Gruppierung steht, worin

$R^4$ für Wasserstoff, Halogen, Alkyl, Halogenalkyl, Alkoxy, Halogenalkoxy, Alkylthio, Alkylamino oder Dialkylamino steht,

dadurch gekennzeichnet, daß man Sulfonylharnstoffe der allgemeinen Formel (II)

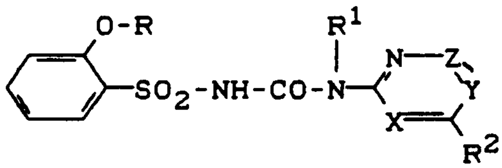

(II)

in welcher

R, $R^1$, $R^2$, X, Y und Z die oben angegebenen Bedeutungen haben,

mit Alkalimetallhydroxiden oder mit basischen organischen Stickstoffverbindungen in Gegenwart von Kohlenwasserstoffen als Verdünnungsmitteln bei Temperaturen zwischen 0 °C und 100 °C umsetzt und die hierbei kristallin anfallenden Produkte nach üblichen Methoden isoliert.

**2.** Verfahren gemäß Anspruch 1 zur Herstellung von Verbindungen der Formel (I), dadurch gekennzeichnet, daß in Formel (I)

$M^{\oplus}$ für ein Lithium-, Natrium- oder Kaliumion, für ein Tri-($C_1$-$C_4$-alkyl)-ammoniumion, für ein N-($C_3$-$C_6$-Cycloalkyl)-N,N-di-($C_1$-$C_4$-alkyl)-ammoniumion oder jeweils für das durch Protonierung gebildete Gegenion von N-($C_1$-$C_4$-Alkyl)-pyrrolidin, N-($C_1$-$C_4$-Alkyl)-piperidin, N-($C_1$-$C_4$-Alkyl)-morpholin, N, N'-Di-($C_1$-$C_4$-alkyl)-piperazin, N,N-Di-($C_1$-$C_4$-alkyl)-benzylamin, 1,5-Diazabicyclo-[4.3.0]-non-5-en (DBN), 1,8-Diazabicyclo-[5.4.0]-undec-7-en (DBU) oder 1,4-Diazabicyclo-[2.2.2]-octan [DABCO] steht,

R für Halogen-$C_1$-$C_4$-alkyl steht,

$R^1$ für $C_1$-$C_6$-Alkyl (welches gegebenenfalls durch Fluor, Cyano, $C_1$-$C_4$-Alkoxy oder $C_1$-$C_4$-Alkylthio substituiert ist), für $C_3$-$C_6$-Alkenyl und $C_3$-$C_6$-Alkinyl (welche gegebenenfalls durch

Fluor oder Chlor substituiert sind) oder für Phenyl-$C_1$-$C_2$-alkyl(welches im Phenylteil gegebenenfalls durch Fluor, Chlor, Nitro, Cyano, Methyl, Methoxy oder $C_1$-$C_2$-Alkoxycarbonyl substituiert ist) steht,

$R^2$ für Wasserstoff, Fluor, Chlor, Brom, Methyl, Ethyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine -CH-Gruppierung steht,

Y für Stickstoff oder eine -$CR^3$-Gruppierung steht, worin $R^3$ für Wasserstoff, Fluor, Chlor, Brom, Methyl, Formyl, Acetyl, Methoxycarbonyl oder Ethoxycarbonyl steht, und

Z für Stickstoff oder eine -$CR^4$-Gruppierung steht, worin

$R^4$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht.

**3.** Verfahren nach Anspruch 1 zur Herstellung von Verbindungen der Formel (I), dadurch gekennzeichnet, daß in Formel (I)

$M^{\oplus}$ für ein Natriumion, ein Kaliumion, für ein Trimethyl-, Triethyl-, Tripropyl oder Tributylammonium ion, für ein N,N-Dimethyl-, N,N-Diethyl-oder N,N-Dipropyl-cyclopentylammoniumion, für ein N,N-Dimethyl-, N,N-Diethyl- oder N,N-Dipropyl-cyclohexylammoniumion, oder jeweils für das durch Protonierung gebildete Gegenion von N-Methyl-, N-Ethyl- oder N-Propyl-pyrrolidin, N-Methyl-, N-Ethyl- oder N-Propyl-piperidin, N-Methyl-, N-Ethyl- oder N-Propyl-morpholin, N,N'-Dimethyl-, N,N'-Diethyl-oder N,N'-Dipropyl-piperazin, N,N-Dimethyl-, N,N-Diethyl- oder N,N-Dipropylbenzylamin, 1,5-Diazabicyclo-[4.3.0]-non-5-en (DBN), 1,8-Diazabicyclo-[5.4.0]- undec-7-en (DBU) oder 1,4-Diazabicyclo-[2.2.2]-octan (DABCO) steht,

R für Difluormethyl oder Trifluormethyl steht,

$R^1$ für Methyl steht,

$R^2$ für Wasserstoff, Chlor, Methyl, Ethyl, Trifluormethyl, Methoxy, Ethoxy, Difluormethoxy, Methylthio, Ethylthio, Methylamino, Ethylamino oder Dimethylamino steht,

X für Stickstoff steht,

Y für Stickstoff oder eine CH-Gruppierung steht und

Z für eine C-$R^4$-Gruppierung steht, worin

$R^4$ für Wasserstoff, Chlor, Methyl, Ethyl, Trifluormethyl, Methoxy, Ethoxy, Difluormethoxy, Methylthio, Ethylthio, Methylamino, Ethylamino oder Dimethylamino steht.

**4.** Sulfonylharnstoff-Salze der Formel (Ia),

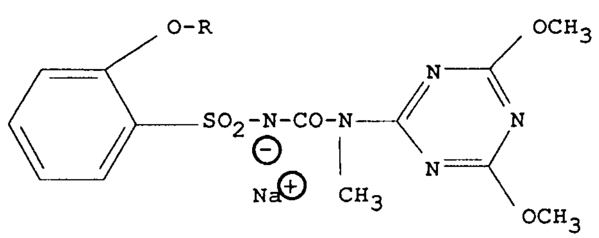

(Ia)

in welcher R für $CF_3$ oder $CHF_2$ steht.

**5.** Herbizide Mittel, gekennzeichnet durch einen Gehalt an mindestens einem Sulfonylharnstoff-Salz der Formel (Ia) gemäß Anspruch 4.

**6.** Verwendung von Sulfonylharnstoff-Salzen der Formel (Ia) gemäß Anspruch 4 zur Bekämpfung von Unkräutern.

**7.** Verfahren zur Herstellung von herbiziden Mitteln, dadurch gekennzeichnet, daß man Sulfonylharnstoff-Salze der Formel (Ia) gemäß Anspruch 4 mit Streckmitteln und/oder oberflächenaktiven Mitteln vermischt.

## Claims

**1.** Process for preparing sulphonylurea salts of the general formula (I)

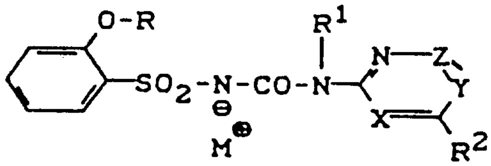

(I)

in which

- $M^{\oplus}$ represents an alkali metal ion or the counterion, formed by protonation, of a basic organic nitrogen compound,
- R represents halogenoalkyl,
- $R^1$ represents optionally substituted radicals from the series comprising alkyl, alkenyl, alkinyl or aralkyl,
- $R^2$ represents hydrogen, halogen, alkyl, halogenoalkyl, alkoxy, halogenoalkoxy, alkylthio, halogenoalkylthio, amino, alkylamino or dialkylamino,
- X represents nitrogen or a -CH group,
- Y represents nitrogen or a $-CR^3$ group where
- $R^3$ represents hydrogen, halogen, cyano, alkyl, formyl, alkyl-carbonyl or alkoxycarbonyl, and
- Z represents nitrogen or a $-CR^4$ group where
- $R^4$ represents hydrogen, halogen, alkyl, halogenoalkyl, alkoxy, halogenoalkoxy, alkylthio, alkylamino or dialkylamino,

characterized in that sulphonylureas of the general formula (II)

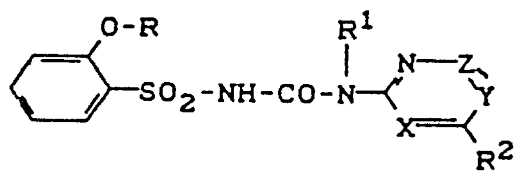

(II)

in which

R, $R^1$, $R^2$, X, Y and Z have the abovementioned meanings

are reacted with alkali metal hydroxides or with basic organic nitrogen compounds in the presence of hydrocarbons as diluents at temperatures between 0 °C and 100 °C, and the products which are obtained in crystalline form in this process are isolated by customary methods.

**2.** Process according to Claim 1 for preparing compounds of the formula (I), characterized in that, in formula (I),

- $M^{\oplus}$ represents a lithium, sodium or potassium ion, a tri-($C_1$-$C_4$-alkyl)-ammonium ion, a N-($C_3$-$C_6$-cycloalkyl)-N,N-d-($C_1$-$C_4$-alkyl)-ammonium ion or in each case the counterion, formed by protonation, of N-($C_1$-$C_4$-alkyl)-pyrrolidine, N-($C_1$-$C_4$-alkyl)-piperidine, N-($C_1$-$C_4$-alkyl)-morpholine, N,N'-di-($C_1$-$C_4$-alkyl)-piperazine, N,N-d-($C_1$-$C_4$-alkyl)-benzylamine, 1,5-diazabicyclo-[4.3.0]-non-5-ene (DBN), 1,8-diazabicyclo-[5.4.0]-undec-7-ene (DBU) or 1,4-diazabicyclo-[2.2.2]-octane [DABCO],
- R represents halogeno-$C_1$-$C_4$-alkyl,
- $R^1$ represents $C_1$-$C_6$-alkyl (which is optionally substituted by fluorine, cyano, $C_1$-$C_4$-alkoxy or $C_1$-$C_4$-alkylthio), or represents $C_3$-$C_6$-alkenyl and $C_3$-$C_6$-alkinyl (which are optionally substituted by fluorine or chlorine), or represents phenyl-$C_1$-$C_2$-alkyl (which is optionally substituted in the phenyl moiety by fluorine, chlorine, nitro, cyano, methyl, methoxy or $C_1$-$C_2$-alkoxycarbonyl),
- $R^2$ represents hydrogen, fluorine, chlorine, bromine, methyl, ethyl, trifluoromethyl, methoxy, ethoxy, difluoromethoxy, methylthio, ethylthio, amino, methylamino, ethylamino,

dimethylamino or diethylamino,

X represents nitrogen or a -CH group,

Y represents nitrogen or a $-CR^3$ group where

$R^3$ represents hydrogen, fluorine, chlorine, bromine, methyl, formyl, acetyl, methoxycarbonyl or ethoxycarbonyl,

and

Z represents nitrogen or a $-CR^4$ group where

$R^4$ represents hydrogen, fluorine, chlorine, bromine, methyl, ethyl, trifluoromethyl, methoxy, ethoxy, propoxy, isopropoxy, difluoromethoxy, methylthio, ethylthio, methylamino, ethylamino, dimethylamino or diethylamino.

**3.** Process according to Claim 1 for preparing compounds of the formula (I), characterized in that, in formula (I),

$M^{\oplus}$ represents a sodium ion or a potassium ion, or represents a trimethyl-, triethyl-, tripropyl- or tributylammonium ion, or a N,N-dimethyl-, N,N-diethyl- or N,N-dipropyl-cyclopentylammnonium ion, a N,N-dimethyl-, N,N-diethyl- or N,N-dipropyl-cyclohexylammonium ion, or in each case the counterion, formed by protonation, of N-methyl-, N-ethyl- or N-propyl-pyrrolidine, N-methyl-, N-ethyl- or N-propyl-piperidine, N-methyl-, N-ethyl- or N-propyl-morpholine, N,N'-dimethyl-, N,N'-diethyl- or N,N'-dipropylpiperazine, N,N-dimethyl-, N,N-diethyl- or N,N-dipropyl-benzylamine, 1,5-diazabicyclo-[4.3.0]-non-5-ene (DBN), 1,8-diazabicyclo-[5.4.0]-undec-7-ene (DBU) or 1,4-diazabicyclo-[2.2.2]-octane (DABCO), or represents a trimethyl-, triethyl-, tripropyl-or tributyl-ammonium ion,

R represents difluoromethyl or trifluoromethyl,

$R^1$ represents methyl,

$R^2$ represents hydrogen, chlorine, methyl, ethyl, trifluoromethyl, methoxy, ethoxy, difluoromethoxy, methylthio, ethylthio, methylamino, ethylamino or dimethylamino,

X represents nitrogen,

Y represents nitrogen or a CH group, and

Z represents a $C-R^4$ group where

$R^4$ represents hydrogen, chlorine, methyl, ethyl, trifluoromethyl, methoxy, ethoxy, difluoromethoxy, methylthio, ethylthio, methylamino, ethylamino or dimethylamino.

**4.** Sulphonylurea salts of the formula (Ia)

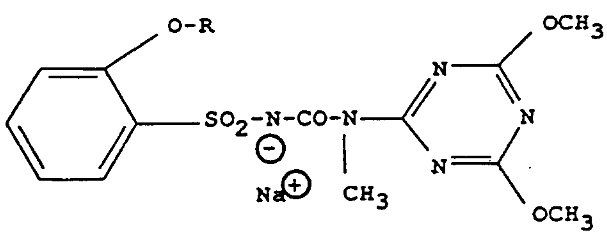

(Ia)

in which R represents $CF_3$ or $CHF_2$.

**5.** Herbicidal agents, characterized in that they contain at least one sulphonylurea salt of the formula (Ia) according to Claim 4.

**6.** Use of sulphonylurea salts of the formula (Ia) according to Claim 4 for combating weeds.

**7.** Process for preparing herbicidal agents, characterized in that sulphonylurea salts of the formula (Ia) according to Claim 4 are mixed with extenders and/or surface-active agents.

## Revendications

**1.** Procédé pour la préparation de sels de sulfonylurées, répondant à la formule générale (I)

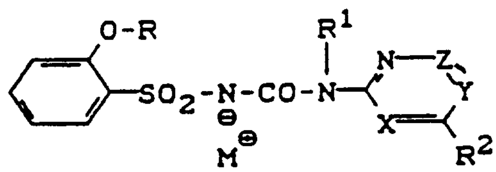

(I)

dans laquelle

- $M^{\oplus}$ représente un ion de métal alcalin ou encore le contre-ion formé par protonation, d'un composé azoté organique basique,
- R représente un groupe halogénalkyle,
- $R^1$ représente des radicaux de la série alkyle, alcényle, alcynyle ou aralkyle, chacun de ces radicaux portant éventuellement un ou plusieurs substituants identiques ou différents,
- $R^2$ représente un atome d'hydrogène, un atome d'halogène, un groupe alkyle, un groupe halogénalkyle, un groupe alcoxy, un groupe halogénalcoxy, un groupe alkylthio, un groupe halogénalkylthio, un groupe amino, un groupe alkylamino ou un groupe dialkylamino,
- X représente un atome d'azote ou un groupement -CH,
- Y représente un atome d'azote ou un groupement $-CR^3$ où
- $R^3$ représente un atome d'hydrogène, un atome d'halogène, un groupe cyano, un groupe alkyle, un groupe formyle, un groupe alkylcarbonyle ou un groupe alcoxycarbonyle,

et

- Z représente un atome d'azote ou un groupement $-CR^4$ où
- $R^4$ représente un atome d'hydrogène, un atome d'halogène, un groupe alkyle, un groupe halogénalkyle, un groupe alcoxy, un groupe halogénalcoxy, un groupe alkylthio, un groupe alkylamino ou un groupe dialkylamino,

caractérisé en ce qu'on fait réagir des sulfonylurées répondant à la formule générale (II)

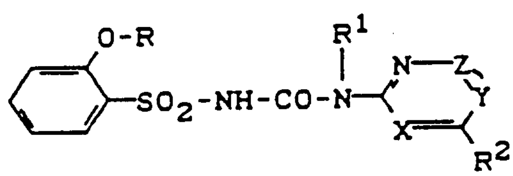

(II)

dans laquelle

R, $R^1$, $R^2$, X, Y et Z ont les significations indiquées ci-dessus,

avec des hydroxydes de métaux alcalins ou avec des composes azotés organiques basiques, en présence d'hydrocarbures comme diluants, à des températures entre 0 °C et 100 °C, et on isole les produits que l'on obtient en l'occurrence sous forme cristalline, conformément à des procédés habituels.

**2.** Procédé selon la revendication 1, pour la préparation de composés de formule (I), caractérisé en ce que, dans la formule (I)

- $M^{\oplus}$ représente un ion lithium, sodium ou potassium, un ion trialkyl(en $C_1$-$C_4$)ammonium, un ion N-cycloalkyl(en $C_3$-$C_6$)-N,N-dialkyl(en $C_1$-$C_4$)ammonium ou encore, respectivement, le contre-ion formé par protonation, de la N-alkyl(en $C_1$-$C_4$)pyrrolidine, de la N-alkyl(en $C_1$-$C_4$)-pipéridine, de la N-alkyl(en $C_1$-$C_4$)morpholine, de la N,N'-dialkyl(en $C_1$-$C_4$)pipérazine, de la N,N-dialkyl(en $C_1$-$C_4$)benzylamine, du 1,5-diazabicyclo-[4.3.0]-non-5-ène (DBN), du 1,8-diazabicyclo-[5.4.0]-undéc-7-ène (DBU) ou du 1,4-diazabicyclo-[2.2.2]-octane (DABCO),
- R représente un groupe halogénalkyle en $C_1$-$C_4$,

$R^1$ représente un groupe alkyle en $C_1$-$C_6$ (qui peut éventuellement porter un ou plusieurs substituants identiques ou différents fluoro, cyano, alcoxy en $C_1$-$C_4$ ou alkyl(en $C_1$-$C_4$)thio), un groupe alcényle en $C_3$-$C_6$ et un groupe alcynyle en $C_3$-$C_6$ (qui peuvent éventuellement porter un ou plusieurs substituants identiques ou différents fluoro ou chloro) ou encore un groupe phénylalkyle en $C_1$-$C_2$ (qui peut éventuellement porter, dans la fraction phényle, un ou plusieurs substituants identiques ou différents fluoro, chloro, nitro, cyano, méthyle, méthoxy ou alcoxy (en $C_1$-$C_2$) carbonyle),

$R^2$ représente un atome d'hydrogène, un atome de fluor, un atome de chlore, un atome de brome, un groupe méthyle, un groupe éthyle, un groupe trifluorométhyle, un groupe méthoxy, un groupe éthoxy, un groupe difluorométhoxy, un groupe méthylthio, un groupe éthylthio, un groupe amino, un groupe méthylamino, un groupe éthylamino, un groupe diméthylamino ou un groupe diéthylamino,

X représente un atome d'azote ou un groupement -CH,

Y représente un atome d'azote ou un groupement -$CR^3$ où

$R^3$ représente un atome d'hydrogène, un atome de fluor, un atome de chlore, un atome de brome, un groupe méthyle, un groupe formyle, un groupe acétyle, un groupe méthoxycarbonyle ou un groupe éthoxycarbonyle,

et

Z représente un atome d'azote ou un groupement -$CR^4$ où

$R^4$ représente un atome d'hydrogène, un atome de fluor, un atome de chlore, un atome de brome, un groupe méthyle, un groupe éthyle, un groupe trifluorométhyle, un groupe méthoxy, un groupe éthoxy, un groupe propoxy, un groupe isopropoxy, un groupe difluorométhoxy, un groupe méthylthio, un groupe éthylthio, un groupe méthylamino, un groupe éthylamino, un groupe diméthylamino ou un groupe diéthylamino.

**3.** Procédé selon la revendication 1, pour la préparation de composés de formule (I), caractérisé en ce que, dans la formule (I),

$M^{\oplus}$ représente un ion sodium, un ion potassium, un ion triméthyl-, triéthyl-, tripropyl- ou tributylammonium, un ion N,N-diméthyl-, N,N-diéthyl- ou N,N-dipropyl-cyclopentylammonium, un ion N,N-diméthyl-, N,N-diéthyl- ou N,N-dipropyl-cyclohexylammonium ou encore, respectivement, le contre-ion formé par protonation, de la N-méthyl-, N-éthyl- ou N-propyl-pyrrolidine, de la N-méthyl-, N-éthyl- ou N-propyl-pipéridine, de la N-méthyl-, N-éthyl- ou N-propyl-morpholine, de la N,N'-diméthyl-, N,N'-diéthyl- ou N,N'-dipropyl-pipérazine, de la N,N-diméthyl-, N,N-diéthyl- ou N,N-dipropyl-benzylamine, du 1,5-diazabicyclo-[4.3.0]-non-5-ène (DBN), du 1,8-diazabicyclo-[5.4.0]-undéc-7-ène (DBU) ou du 1,4-diazabicyclo-[2.2.2]-octane (DABCO),

R représente un groupe difluorométhyle ou trifluorométhyle,

$R^1$ représente un groupe méthyle,

$R^2$ représente un atome d'hydrogène, un atome de chlore, un groupe méthyle, un groupe éthyle, un groupe trifluorométhyle, un groupe méthoxy, un groupe éthoxy, un groupe difluorométhoxy, un groupe méthylthio, un groupe éthylthio, un groupe méthylamino, un groupe éthylamino ou un groupe diméthylamino,

X représente un atome d'azote,

Y représente un atome d'azote ou un groupement -CH, et

Z représente un groupement -$CR^4$ où

$R^4$ représente un atome d'hydrogène, un atome de chlore, un groupe méthyle, un groupe éthyle, un groupe trifluorométhyle, un groupe méthoxy, un groupe éthoxy, un groupe difluorométhoxy, un groupe méthylthio, un groupe éthylthio, un groupe méthylamino, un groupe éthylamino ou un groupe diméthylamino.

4. Sels de sulfonylurées répondant à la formule (Ia)

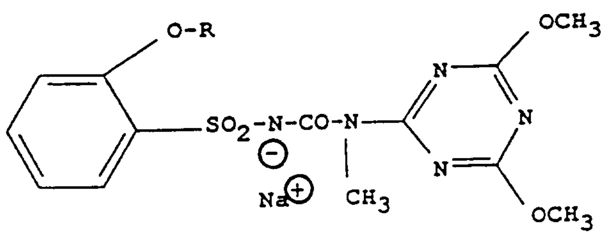

(Ia)

dans laquelle R représente $CF_3$ ou $CHF_2$.

5. Agents herbicides caractérisés par une teneur en au moins un sel de sulfonylurée de formule (Ia) selon la revendication 4.

6. Utilisation de sels de sulfonylurées de formule (Ia) selon la revendication 4, pour lutter contre les mauvaises herbes.

7. Procédé pour la préparation d'agents herbicides, caractérisé en ce qu'on mélange des sels de sulfonylurées de formule (Ia) selon la revendication 4 avec des diluants et/ou des agents tensioactifs.